(19)

Europäisches
Patentamt
European
Patent Office
Office européen
des brevets

(11) **EP 3 865 151 A1**

(12) **EUROPEAN PATENT APPLICATION**
published in accordance with Art. 153(4) EPC

(43) Date of publication:
**18.08.2021 Bulletin 2021/33**

(21) Application number: **19871771.2**

(22) Date of filing: **10.10.2019**

(51) Int Cl.:
*A61K 39/395* (2006.01)   *A61K 45/00* (2006.01)
*A61K 47/65* (2017.01)   *A61K 47/68* (2017.01)
*A61P 35/00* (2006.01)   *A61P 35/02* (2006.01)
*B01J 19/00* (2006.01)   *C07K 16/18* (2006.01)

(86) International application number:
**PCT/JP2019/040059**

(87) International publication number:
**WO 2020/075817 (16.04.2020 Gazette 2020/16)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **10.10.2018  JP 2018191486**

(71) Applicant: **Takeda Pharmaceutical Company
Limited
Osaka-shi, Osaka 541-0045 (JP)**

(72) Inventors:
• **SUENAGA Masato**
  **Hikari-shi, Yamaguchi 743-0011 (JP)**
• **TAKENAKA Kosuke**
  **Hikari-shi, Yamaguchi 743-0011 (JP)**
• **IWAMOTO Keiji**
  **Hikari-shi, Yamaguchi 743-0011 (JP)**

(74) Representative: **Huenges, Martin
Maiwald Patentanwalts- und
Rechtsanwaltsgesellschaft mbH
Elisenhof
Elisenstraße 3
80335 München (DE)**

(54)   **METHOD FOR MANUFACTURING ANTIBODY-DRUG CONJUGATE**

(57)    The present invention provides a method for producing an antibody-drug conjugate (ADC) comprising an antibody and a drug linked to each other via a linker. The present invention provides a method for producing, for example, an antibody-drug conjugate (ADC) comprising an antibody and a drug linked to each other via a linker, or a pharmaceutical comprising the ADC, the method comprising mixing, using a microreactor, a solution comprising tricarboxyethyl phosphine (TCEP) and an IgG antibody under reduction reaction with TCEP, with a solution comprising a stoichiometrically excessive amount of an inhibitor of TCEP based on TCEP.

FIG. 2

MICROREACTOR METHOD OF EXAMPLE 5

## Description

[Technical Field]

The present invention relates to a method for producing an antibody-drug conjugate.

Background Art

A monoclonal antibody is useful as target therapy for diseases such as cancer. For purposes of enhancing cytotoxicity of a monoclonal antibody against a cancer cell or the like, an antibody-drug conjugate (ADC) comprising a monoclonal antibody and a drug (such as a cytotoxic drug) linked to each other has been developed (Patent Literature 1 and the like).
In most of ADCs used in clinical trials, an average of an averaged number of drugs bound to an antibody molecule (drug to antibody ratio; DAR) is 3 to 4, and this number is presumed as an optimal number (Non Patent Literature 1).

[Citation List]

[Patent Literature]

[Patent Literature 1] International Publication No. WO2005/117986

[Non Patent Literature]

[Non Patent Literature 1] Debaene F. et al., Analytical Chemistry, 86: 10674-10683, 2014

[Brief Description of Invention]

The present invention provides a method for producing an antibody-drug conjugate.
According to the present invention, in preparation process of an antibody-drug conjugate (ADC), when an inhibitor of tricarboxyethyl phosphine (TCEP) is mixed, using a microreactor, with an antibody reduced with TCEP, a drug to antibody ratio (DAR) in the ADC can be controlled, and thus, the ADC with the DAR controlled may be produced. According to the present invention, particularly in preparation process of an antibody-drug conjugate (ADC), an antibody and tricarboxyethyl phosphine (TCEP) are mixed for reduction using a microreactor, and thereafter, an inhibitor of TCEP is mixed, using a microreactor, with the antibody under reduction with tricarboxyethyl phosphine (TCEP), and thus, a drug to antibody ratio (DAR) in the ADC can be controlled, and the ADC with the DAR controlled may be produced.
The present invention may provide, for example, the following inventions:

(1) A method for producing an antibody-drug conjugate (ADC) comprising an antibody and a drug linked to each other via a linker, or a pharmaceutical comprising the ADC, the method comprising
mixing, using a microreactor, a solution comprising a reducing agent for reducing a disulfide bond of the antibody and a partially reduced IgG antibody under reduction reaction with the reducing agent, with a solution comprising an inhibitor of the reducing agent and/or a reduction terminator.
(2) A method for producing an antibody-drug conjugate (ADC) comprising an antibody and a drug linked to each other via a linker, or a pharmaceutical comprising the ADC, the method comprising:

(b) mixing, using a microreactor, a solution comprising tricarboxyethyl phosphine (TCEP) and a partially reduced IgG antibody under reduction reaction with TCEP, with a solution comprising a stoichiometrically excessive amount of an inhibitor of TCEP based on TCEP.

(3) The method according to (2) described above, further comprising:

(a) mixing, using a microreactor, a solution comprising an IgG antibody with a solution comprising tricarboxyethyl phosphine (TCEP) to generate a partially reduced antibody.

(4) The method according to (2) or (3) described above, further comprising:

(c) reacting the partially reduced antibody with a linker having a functional group reactive with an SH group of the antibody to generate the antibody linked to the linker.

(5) A method for producing an antibody-drug conjugate (ADC) comprising an antibody and a drug linked to each other via a linker, or a pharmaceutical comprising the ADC, the method comprising:

(a) mixing, using a microreactor, a solution comprising an IgG antibody with a solution comprising tricarboxyethyl phosphine (TCEP) to generate a partially reduced antibody;
(b) mixing, using a microreactor, a solution comprising tricarboxyethyl phosphine (TCEP) and the partially reduced IgG antibody under reduction reaction with TCEP, with a solution comprising a stoichiometrically excessive amount of an inhibitor of TCEP based on TCEP; and
(c) reacting the partially reduced antibody with a linker having a functional group reactive with an SH group of the antibody to generate the antibody linked to the linker.

(5') The method for producing an antibody-drug conjugate (ADC) comprising an antibody and a drug linked to each other via a linker, or a pharmaceutical comprising the ADC according to any one of (1) to (4) described above, comprising:

(a) mixing, using a microreactor, a solution comprising an IgG antibody with a solution comprising tricarboxyethyl phosphine (TCEP) to generate a partially reduced antibody;
(b) mixing, using a microreactor, a solution comprising tricarboxyethyl phosphine (TCEP) and the partially reduced IgG antibody under reduction reaction with TCEP with a solution comprising a stoichiometrically excessive amount of an inhibitor of TCEP based on TCEP; and
(c) reacting the partially reduced antibody with a linker having a functional group reactive with an SH group of the antibody to generate the antibody linked to the linker.

(6) The method according to any one of (2) to (5) described above, wherein the solution comprising the inhibitor of TCEP further comprises the linker.
(7) The method according to (4) or (5) described above, further comprising, before (c), mixing, using a microreactor, a solution obtained in (b) with a solution comprising the linker.
(8) The method according to any one of (2) to (7) described above, wherein the inhibitor of TCEP is one or more inhibitors selected from the group consisting of 4-azidobenzoic acid and 2-azidoethyl-2-acetamide-2-deoxy-β-D-glucopyranoside.
(9) The method according to any one of (2) to (8) described above, wherein the partially reduced antibody is an antibody having four SH groups.
(10) The method according to any one of (4) to (9) described above, wherein the functional group reactive with an SH group of the antibody is a maleimide group.
(11) The method according to any one of (4) to (10) described above, wherein the linker is a linker linked to a drug.
(12) The method according to any one of (4) to (11) described above, wherein the linker is linked to a drug, and the functional group reactive with an SH group of the antibody is a maleimide group.

[Brief Description of Drawings]

[Figure 1] Figure 1 illustrates chromatographs of antibody-drug conjugates (ADCs) obtained by production methods of Examples 1 to 3. In this drawing, DAR denotes a drug to antibody ratio (namely, a number of drugs bound to antibody). For example, DAR0 denotes a peak of an ADC having a drug to antibody ratio of 0.
[Figure 2] Figure 2 illustrates a chromatograph of an ADC obtained by a production method of Example 5.
[Figure 3] Figure 3 illustrates chromatographs of ADCs obtained by production methods of Example 6 and Example 7.
[Figure 4] Figure 4 illustrates chromatographs of ADCs obtained by production methods of Example 7 and Example 8.
[Figure 5] Figure 5 illustrates chromatographs of ADCs obtained by production methods of Example 8 and Example 9.

[Detailed Description of Invention]

The term "microreactor" as used herein refers to a flow reactor equipped with a channel applicable to a liquid phase. The microreactor may include a channel having a representative diameter of 1 mm or less (for example, possibly having a width and a depth both of 1 mm or less). In the microreactor, a plurality of (for example, two) channels (namely, supply channels for chemical substances) are joined to one reaction channel for mixing compounds within the reaction channel, and thus, a reaction can be started. After starting the reaction using the microreactor, the reaction can be caused to further proceed in the microreactor, or out of the microreactor (for example, in a tube externally extending from the reaction channel). The liquid phase to be applied to the microreactor may be filtered so as to prevent clogging of the channel otherwise caused through introduction of a solution.

The term "antibody" as used herein means an immunoglobulin. Examples of the antibody include antibodies of various animals, a human antibody, a human chimeric antibody, and a humanized antibody. Examples of the antibody include a polyclonal antibody and a monoclonal antibody. Examples of the antibody include a monospecific antibody and a bispecific antibody. In an antibody-drug conjugate, a monoclonal antibody may preferably be used. The antibody includes an antigen binding fragment (for example, one having the same cysteine number as the original antibody). Herein, a full length antibody is referred to as an intact antibody in some cases.

A human antibody can be obtained by, for example, antigen immunization in an animal (for example, a mouse) produced by replacing an antibody gene locus of the animal with a human antibody gene locus. A humanized antibody may be obtained by grafting a complementarity determining region of an antibody obtained from an animal onto a human antibody. A human chimeric antibody may be obtained by replacing variable regions of a human antibody with a heavy chain variable region and a light chain variable region of an antibody obtained from an animal.

A monoclonal antibody may be obtained, for example, from a hybridoma strain obtained by forming a hybridoma through fusion of a myeloma cell with an antibody producing cell, and cloning the resultant.

Examples of the antigen binding fragment include Fab, Fab', $F(ab')_2$, a half antibody (rIgG), and scFv. The antigen binding fragment may be obtained through a treatment for fragmenting an antibody (for example, a treatment with peptidase such as papain or pepsin), or reduction of a disulfide bond.

The term "antibody-drug conjugate" as used herein means a conjugate in which a drug (for example, a cytotoxic drug) is linked to an antibody via or without a linker by a covalent bond. Herein, an antibody-drug conjugate is sometimes referred to simply as an "ADC". The antibody used in an ADC can be an IgG antibody. Examples of the IgG antibody include IgG1, IgG2, IgG3, and IgG4, which may be used in an ADC. Herein, an antibody not linked to a drug is referred to as a "naked antibody" in some cases. Herein, an antibody means a naked antibody unless otherwise specified.

An IgG antibody consists of two heavy chains and two light chains, the heavy chains and the light chains form a disulfide bond (in one position) between the cysteine residues thereof, and in the two heavy chains, the disulfide bond between the cysteine residues may be present, for example, in two positions in IgG1 and IgG4, four positions in IgG2, and eleven positions in IgG3. In producing an ADC, the disulfide bond is cleaved by a reducing agent to generate an SH group, and a drug and the antibody may be linked to each other via a linker having a functional group reactive with the SH group. In the IgG antibody, the heavy chain may have 4 intrachain sulfide bonds, and the light chain may have 2 intrachain sulfide bonds.

Accordingly, the ADC may be represented by the following formula (I):

$$\text{Antibody} - \{L - D\}m \quad (I),$$

wherein L represents a linker, D represents a drug, and m may represent an integer of 1 to 8. Herein, m may correspond to a drug to antibody ratio (DAR).

In the ADC, a linker (a cleavable linker or a non-cleavable linker) having a functional group reactive with an SH group of the antibody may be used as the linker. An example of the functional group reactive with an SH group includes a maleimide group. Accordingly, in the present invention, the antibody and a linker having a maleimide group may be reacted with each other. Examples of the cleavable linker include a linker having a hydrazone bond, and a linker having a cleavage site for protease (for example, a linker having a cathepsin B cleavage site, a cathepsin C cleavage site or a cathepsin D cleavage site), and Gly-Gly, Phe-Lys, Val-Lys, Phe-Phe-Lys, D-Phe-Phe-Lys, Gly-Phe-Lys, Ala-Lys, Val-Cit, Phe-Cit, Leu-Cit, Ile-Cit, Trp-Cit, Phe-Ala, Ala-Phe, Gly-Gly-Gly, Gly-Ala-Phe, Gly-Val-Cit, Gly-Phe-Leu-Gly, Ala-Leu-Ala-Leu, Phe-N[9]-tosyl-Arg, and Phe-N[9]-nitro-Arg may be used {wherein three-letter codes for the amino acids are used in general meaning thereof}. Besides, examples of the linker include maleimide caproyl; maleimide caproyl-p-aminobenzylcarbamate; maleimide caproyl-peptide-aminobenzylcarbamate (wherein peptide may be a cleavage site for peptidase, such as maleimide caproyl-L-phenylalanine-L-lysine-p-aminobenzylcarbamate and maleimide caproyl-L-valine-L-citrulline-p-aminobenzylcarbamate (vc)); N-[β-maleimide propyloxy]succinimide ester (BMPS); [N-ε-maleimide caproyloxy]succinimide ester (EMCS); N[γ-maleimide butyloxy]succinimide ester (GMBS); m-maleimide benzoyl-N-hydroxysuccinimide ester (MBS); [N-ε-maleimide caproyloxy]sulfosuccinimide ester (sulfo-EMCS); N-[γ-maleimide butylyloxy]sulfosuccinimide ester (sulfo-GMBS); and m-maleimide benzoyl-N-hydroxysulfosuccinimide ester (sulfo-MBS), which may be used as a linker in the present invention.

Examples of the drug include an anticancer agent and a chemotherapeutic agent (for example, an agent inhibiting onset or progression of a neoplasm in a human (particularly, lesion such as carcinoma, sarcoma, lymphoma, or leukemia); an agent inhibiting metastasis of a neoplasm or neovascularization; a cytotoxic drug; or a cytostatic (an agent inhibiting or suppressing cell growth and/or cell proliferation)).

Examples of the cytotoxic drug or the cytostatic may include an antimetabolite (for example, azathioprine, 6-mercaptopurine, 6-thioguanine, fludarabine, pentostatin, cladribine, 5-fluorouracil (5FU), floxuridine (FUDR), cytosine arabinoside (cytarabine), methotrexate, trimethoprim, pyrimethamine, or pemetrexed); an alkylating agent (for example, cyclophosphamide, mechlorethamine, uramustine, melphalan, chlorambucil, thiotepa/chlorambucil, ifosfamide, carmustine, lomustine, streptozocin, busulfan, dibromomannitol, cisplatin, carboplatin, nedaplatin, oxaliplatin, satraplatin, triplatin tetranitrate, procarbazine, altretamine, dacarbazine, mitozolomide, or temozolomide); an anthracycline (for example, daunorubicin, doxorubicin, epirubicin, idarubicin, or valrubicin); an antibiotic (for example, dactinomycin, bleomycin, mithramycin, anthramycin, streptozotocin, gramicidin D, a mitomycin (such as mitomycin C), a duocarmycin (such as CC-1065), or a calicheamicin); a mitotic inhibitor (a maytansinoid (such as maytansine), auristatin (such as auristatin E, auristatin phenylalanine phenylenediamine (AFP), monomethyl auristatin E, monomethyl auristatin D, and monomethyl auristatin F), a dolastatin, a cryptophycin, vinca alkaloid (for example, vincristine, vinblastine, vindesine, or vinorelbine), a taxane (for example, paclitaxel, docetaxel, or a novel taxane (see, for example, International Publication No. WO01/38318), or a colchicine; a topoisomerase inhibitor (for example, irinotecan, topotecan, amsacrine, etoposide, teniposide, or mixantrone); and a proteasome inhibitor (for example, peptidylboronic acid); or a pharmaceutically acceptable salt (specifically, a salt of the same type as a salt described as a specific example of a salt of histidine below) of any of these.

As a therapeutic agent, a mitotic agent is preferred; a maytansinoid or auristatin is more preferred; maytansine or auristatin (particularly, monomethyl auristatin) is further preferred; monomethyl auristatin E (herein also referred to as MMAE) or monomethyl auristatin D (herein also referred to as MMAD) is further more preferred.

The present invention provides a method for producing an antibody-drug conjugate (ADC) comprising an antibody and a drug linked to each other via a linker, or a pharmaceutical comprising the ADC, the method comprising mixing, using a microreactor, a solution comprising a reducing agent for reducing a disulfide bond of an IgG antibody and a partially reduced IgG antibody under reduction reaction with the reducing agent, with a solution comprising a stoichiometrically excessive amount of an inhibitor (or a reduction reaction terminator) based on the reducing agent. This method may further comprise mixing a solution comprising an IgG antibody and a solution comprising the reducing agent for reducing a disulfide bond of the IgG antibody to generate a partially reduced antibody. This method may further comprise reacting the partially reduced antibody with a linker having a functional group reactive with an SH group of the antibody to generate the antibody linked to the linker. Examples of the reducing agent include various reducing agents such as tricarboxyethyl phosphine (TCEP), 2-mercaptoethanol, 2-mercaptoethylamine, cysteine hydrochloride, dithiothreitol, and a salt (such as hydrochloride) of any of these, which can be used for reducing a disulfide bond. As the inhibitor, an inhibitor against each of these reducing agents can be appropriately used. As the reduction reaction terminator, any agent terminating the reduction reaction can be used. In a preferable aspect of the present invention, the reducing agent is TCEP, and the inhibitor is one or more inhibitors selected from the group consisting of 4-azidobenzoic acid and 2-azidoethyl-2-acetamido-2-deoxy-$\beta$-D-glucopyranoside, and in a more preferable aspect, the inhibitor is 2-azidoethyl-2-acetamido-2-deoxy-$\beta$-D-glucopyranoside.

The present invention provides a method for producing an antibody-drug conjugate (ADC) comprising an antibody and a drug linked to each other via a linker, or a pharmaceutical comprising the ADC, the method comprising

(b) mixing, using a microreactor, a solution comprising tricarboxyethyl phosphine (TCEP) and a partially reduced IgG antibody under reduction reaction with TCEP, with a solution comprising a stoichiometrically excessive amount of an inhibitor of TCEP based on TCEP.

According to the present invention, the production method may further comprise

(a) mixing a solution comprising an IgG antibody with a solution comprising tricarboxyethyl phosphine (TCEP) to generate a partially reduced antibody {wherein the mixing may be performed using or not using a microreactor, and can be preferably performed using a microreactor}.

According to the present invention, the production method may further comprise

(c) reacting the partially reduced antibody with a linker having a functional group reactive with an SH group of the antibody to generate the antibody linked to the linker.

Accordingly, the present invention provides a method for producing an antibody-drug conjugate (ADC) comprising an antibody and a drug linked to each other via a linker, or a pharmaceutical comprising the ADC, the method comprising:

EP 3 865 151 A1

(a) mixing a solution comprising an IgG antibody with a solution comprising tricarboxyethyl phosphine (TCEP) to generate a partially reduced antibody {wherein the mixing may be performed using or not using a microreactor, and can be preferably performed using a microreactor};
(b) mixing, using a microreactor, a solution comprising tricarboxyethyl phosphine (TCEP) and the partially reduced IgG antibody under reduction reaction with TCEP with a solution comprising a stoichiometrically excessive amount of an inhibitor of TCEP based on TCEP; and
(c) reacting the partially reduced antibody with a linker having a functional group reactive with an SH group of the antibody to generate the antibody linked to the linker.

In one aspect, the present invention provides a method for producing an antibody-drug conjugate (ADC) comprising an antibody and a drug linked to each other via a linker, or a pharmaceutical comprising the ADC, the method comprising:

(a) mixing a solution comprising an IgG antibody with a solution comprising tricarboxyethyl phosphine (TCEP) to generate a partially reduced antibody {wherein the mixing may be performed using or not using a microreactor, and can be preferably performed using a microreactor};
(b) mixing, using a microreactor, a solution comprising tricarboxyethyl phosphine (TCEP) and the partially reduced IgG antibody under reduction reaction with TCEP with a solution comprising a stoichiometrically excessive amount of an inhibitor of TCEP based on TCEP; and
(c) reacting the partially reduced antibody with a linker having a functional group reactive with an SH group of the antibody to generate the antibody linked to the linker, wherein the partially reduced antibody is an antibody having four SH groups, the inhibitor of TCEP is one or more inhibitors selected from the group consisting of 4-azidobenzoic acid and 2-azidoethyl-2-acetamido-2-deoxy-β-D-glucopyranoside (more preferably, 2-azidoethyl-2-acetamido-2-deoxy-β-D-glucopyranoside), and the functional group reactive with the SH group of the antibody is a maleimide group.

In one aspect, the present invention provides a method for producing an antibody-drug conjugate (ADC) comprising an antibody and a drug linked to each other via a linker, or a pharmaceutical comprising the ADC, the method comprising:

(a) mixing a solution comprising an IgG antibody with a solution comprising tricarboxyethyl phosphine (TCEP) to generate a partially reduced antibody {wherein the mixing may be performed using or not using a microreactor, and can be preferably performed using a microreactor};
(b) mixing, using a microreactor, a solution comprising tricarboxyethyl phosphine (TCEP) and a partially reduced IgG antibody under reduction reaction with TCEP with a solution comprising a stoichiometrically excessive amount of an inhibitor of TCEP based on TCEP; and
(c) reacting the partially reduced antibody with a linker having a functional group reactive with an SH group of the antibody to generate the antibody linked to the linker, wherein the partially reduced antibody is an antibody having four SH groups, the inhibitor of TCEP is one or more inhibitors selected from the group consisting of 4-azidobenzoic acid and 2-azidoethyl-2-acetamido-2-deoxy-β-D-glucopyranoside (more preferably, 2-azidoethyl-2-acetamido-2-deoxy-β-D-glucopyranoside), the solution comprising the inhibitor of TCEP further comprises a linker having a functional group reactive with the SH group of the antibody, the functional group reactive with the SH group of the antibody is a maleimide group, and the linker is linked to one or more drugs in a different portion from the maleimide group (such as a different end from the maleimide group).

Now, the steps (a) to (c) described above, and a step (b') and other steps that may be additionally included in the present invention will be described.

(a) Mixing Solution Comprising IgG Antibody and Solution Comprising Tricarboxyethyl Phosphine (TCEP) to Generate Partially Reduced Antibody

In the step (a), a disulfide bond between cysteine residues linking between peptide chains of an antibody is reduced. As a reducing agent, TCEP reducing a disulfide bond to an SH group can be used.
In the step (a), mixing may be performed using or not using a microreactor, and the mixing is preferably performed using a microreactor. In the step (a), if a microreactor is used, a microreactor including a first supply channel, a second supply channel, and a joining channel joining the supply channels may be used as the microreactor. The channels of the microreactor may be designed to have a representative diameter (such as a width or a depth) of 10 μm to 1 mm, or 100 μm to 1 mm. In the step (a), setting may be performed so as to introduce the solution comprising the IgG antibody through the first supply channel, to introduce the solution comprising tricarboxyethyl phosphine (TCEP) through the second supply channel, and to mix these solutions in the joining channel.

A concentration of the antibody in the solution comprising the antibody may be, for example, 1 mg/mL to 100 mg/mL. An antigen of the antibody is not especially limited.

A concentration of TCEP in the solution comprising TCEP may be, for example, 1 mM to 100 mM.

TCEP may be mixed, for example, in an excessive amount based on the antibody. TCEP can be mixed in an amount of 1 to 50-fold molar equivalent, for example, 2 to 30-fold molar equivalent, for example, 5 to 20-fold molar equivalent, for example, 7 to 13-fold molar equivalent, for example, 4 to 30-fold molar equivalent, or for example, 10-fold equivalent with respect to the antibody. A mixing ratio may be controlled in accordance with the concentrations of the antibody and/or TCEP in the solutions to be mixed, or flow rates.

In the present invention, in the step (a), the partially reduced antibody is obtained.

When the two solutions are mixed to bring the antibody and the reducing agent into contact with each other, the antibody is reduced. The degree of the reduction of the antibody is associated with a drug to antibody ratio in the ADC to be obtained. Since the antibody has four disulfide bonds between chains, complete reduction of these bonds results in eight SH groups. Accordingly, a drug to antibody ratio in the ADC to be obtained by reducing the disulfide bonds between the chains may be an integer in a range of 0 to 8.

The partially reduced antibody may have two to six SH groups. In a preferable aspect of the present invention, the partially reduced antibody may have four SH groups. In a preferable aspect of the present invention, a ratio of the antibody having four SH groups may be, in the whole treated antibody, 30% or more, 31% or more, 32% or more, 33% or more, 34% or more, 35% or more, 36% or more, 37% or more, 38% or more, 39% or more, 40% or more, 41% or more, 42% or more, 43% or more, 44% or more, 45% or more, 46% or more, 47% or more, 48% or more, 49% or more, or 50% or more. In a preferable aspect of the present invention, the ratio of the antibody having four SH groups may be, in the whole treated antibody, 60% or more, 70% or more, 80% or more, or 90% or more.

The reduction of the antibody may be performed in a channel within the microreactor, or may be performed in a channel within a pipe (tube) or a microchannel plate connected to the microreactor. The tube or the microchannel plate can be determined in a flow rate and a length of the channel in accordance with a reaction time. The reduction of the antibody can be performed under heating to an extent that protein is not denatured (for example, to a temperature in a range of room temperature to 37°C). A reduction time may be appropriately adjusted in accordance with the amount of TCEP to be added. Here, a treatment time may be set so as to increase a ratio of the antibody having four SH groups and/or an ADC having a drug to antibody ratio of 4. For example, if TCEP is used in an amount of 10-fold molar equivalent of the antibody, the reduction time may be several seconds to 5 minutes, for example, several seconds to about 2 minutes, preferably 1 minute to 5 minutes, and more preferably about 1 minute to 2 minutes. When the mixing is performed using a microchannel, a time necessary for the mixing is extremely shortened, and hence the reduction reaction of the antibody may be homogeneous, and besides, also when the reduction time is short, the microreactor can be suitably used. Those skilled in the art could appropriately adjust the concentration of TCEP to be used and the reduction time in accordance with a target DAR value that a target ADC should attain.

(b) Mixing, Using Microreactor, Solution Comprising Tricarboxyethyl Phosphine (TCEP) and Partially Reduced IgG Antibody under Reduction Reaction with TCEP with Solution Comprising a Stoichiometrically Excessive Amount of an Inhibitor of TCEP Based on TCEP

Now, the step (b) will be described.

In the present invention, the solution of the partially reduced antibody comprises TCEP mixed in the step (a). In the step (b), the solution comprising tricarboxyethyl phosphine (TCEP) and the partially reduced IgG antibody under reduction reaction with TCEP and the solution comprising a stoichiometrically excessive amount of the inhibitor of TCEP based on TCEP are brought into contact with each other using a microreactor.

The inhibitor of TCEP is not especially limited, and may be, for example, an azide compound or a diazide compound, and examples include various inhibitors of TCEP such as 4-azidobenzoic acid, azide-PEG3-azide, 5-azidopentanoic acid, 2-azidoethyl-2-acetamido-2-deoxy-β-D-glucopyranoside, ethylazidoacetate, and trimethylsilyl azide, which can be used in the present invention. In one aspect of the present invention, the inhibitor of TCEP may be one or more inhibitors selected from the group consisting of 4-azidobenzoic acid, and 2-azidoethyl-2-acetamido-2-deoxy-β-D-glucopyranoside, and in particular, is preferably 2-azidoethyl-2-acetamido-2-deoxy-β-D-glucopyranoside. In another aspect, the inhibitor of TCEP may be 4-azidobenzoic acid.

The inhibitor of TCEP can be in a stoichiometrically excessive amount based on TCEP contained in the solution, and thus, further reduction of the antibody with TCEP may be stopped. The stoichiometrically excessive amount can be, based on TCEP, 2-fold molar equivalent or more, 3-fold molar equivalent or more, 4-fold molar equivalent or more, 5-fold molar equivalent or more, 6-fold molar equivalent or more, 7-fold molar equivalent or more, 8-fold molar equivalent or more, 9-fold molar equivalent or more, or 10-fold molar equivalent or more. A mixing ratio can be controlled in accordance with the concentrations of TCEP and/or the inhibitor in the solutions to be mixed, and flow rates. Thus, the reduction of the antibody may be rapidly stopped.

In another aspect of the present invention, if the inhibitor of TCEP to be added is 4-azidobenzoic acid, the reduction time for the antibody may be 1 minute to 5 minutes, and preferably about 1 minute to 2 minutes. In another aspect of the present invention, if the inhibitor of TCEP to be added is 2-azidoethyl-2-acetamido-2-deoxy-β-D-glucopyranoside, the reduction time for the antibody may be several seconds to 1 minute, preferably several seconds to 20 seconds, and more preferably about 10 seconds. Those skilled in the art could appropriately adjust the concentration of the inhibitor of TCEP to be added and the reaction time. Since the inhibitor of TCEP is used for stopping the reduction of the antibody to fix a reduction state of the antibody at that time, those skilled in the art could appropriately adjust the type and the concentration of the inhibitor to be used and the reaction time.

It is presumed that the concentration of the reducing agent, the reduction time, and the type and the concentration of the inhibitor of the reducing agent are associated with homogeniety of the DAR of the ADC, and it is presumed that the type and the concentration of the reducing agent and the reduction time are associated with the magnitude of the DAR. The reduction reaction may be performed also by mixing an excessive amount of a substrate of the reducing agent with the antibody solution. Such a substrate of the reducing agent may be preferably used as a reduction terminator. In addition, in the present invention, the reduction reaction of the antibody can be stopped by using a reduction terminator known to those skilled in the art. Those skilled in the art would understand that a preferable DAR is varied by using a different drug or antibody. Accordingly, those skilled in the art can perform the step (a) of the present invention suitably for a preferable DAR. Besides, it should be understood that the step (b) of the present invention is preferably performed, in either case, by mixing an excessive amount of the inhibitor for a sufficient time period.

In the step (c) described below, the reduced antibody and a linker are reacted with each other to generate the antibody linked to the linker.

In one aspect of the present invention, a linker (for example, a linker linked to a drug) can be added simultaneously with the inhibitor of TCEP. In this aspect, the solution comprising the inhibitor of TCEP can further comprise the linker. In another aspect of the present invention, the production method may comprise (b2) mixing the solution comprising the antibody with a solution comprising a linker (for example, a linker linked to a drug) using a microreactor, after mixing the inhibitor of TCEP. As the linker, any of the above-described linkers may be used.

The steps (a) and (b) can be performed respectively using different microreactors, and the different microreactors may be incorporated into one chip or substrate, or incorporated into different chips or substrates. If the microreactor used in the step (a) and the microreactor used in the step (b) are incorporated into one chip or substrate, the microreactors may be linked to each other via a channel (wherein the reduction reaction of the antibody may proceed). If the microreactor used in the step (a) and the microreactor used in the step (b) are incorporated into different chips or substrates, the microreactors may be linked to each other via a tube (wherein the reduction reaction of the antibody may proceed).

Similarly, the steps (b) and (b2) can be performed respectively using different microreactors, and the different microreactors may be incorporated into one chip or substrate, or incorporated into different chips or substrates. If the microreactor used in the step (b) and the microreactor used in the step (b2) are incorporated into one chip or substrate, the microreactors may be linked to each other via a channel (wherein the reduction reaction of the antibody may proceed). If the microreactor used in the step (b) and the microreactor used in the step (b2) are incorporated into different chips or substrates, the microreactors may be linked to each other via a tube (wherein the reduction reaction of the antibody may proceed).

From the viewpoint of increasing throughput, at least one of or all of the steps (a), (b) and (b2) may be performed in parallel. Besides, the steps (a), (b) and (b2) may be performed in series using microreactors linked to each other in tandem.

(c) Reacting Partially Reduced Antibody with Linker having Functional Group Reactive with SH group of Antibody to Generate Antibody linked to Linker

In the step (c), the partially reduced antibody (namely, the antibody having an SH group) and the linker having a functional group reactive with SH of the antibody are reacted with each other. Thus, an antibody linked to the linker may be generated. The linker in, for example, a stoichiometrically excessive amount based on the number of SH groups of the reduced antibody may be brought into contact with the antibody.

In the step (c), the reduced antibody and the linker are reacted with each other to generate the antibody linked to the linker.

In one aspect of the present invention, a linker (for example, a linker linked to a drug) can be added simultaneously with the inhibitor of TCEP in the step (b). In this aspect, the solution comprising the inhibitor of TCEP can further comprise the linker.

In another aspect of the present invention, the production method may comprise (b2) mixing, using a microreactor, a solution comprising the antibody with a solution comprising a linker (for example, a linker linked to one or more drugs) after mixing the inhibitor of TCEP. Any of the above-described linkers may be used as the linker.

The steps (a), (b) and (c) are performed in the stated order, or may be performed in the stated order. The steps excluding the step (b) may be performed without using a microreactor. For example, the steps (a), (c) or (a) and (c) may be performed without using a microreactor. The structure of the microreactor has been described above regarding the step (a).

The linker may not be linked to a drug, and may be preferably linked to a drug. When the partially reduced antibody, for example, the antibody having four SH groups after inhibiting the reduction reaction is reacted with an excessive amount of the linker, an antibody having a drug to antibody ratio of 4 may be produced in a large amount. If the linker is not linked to a drug, the linker may have a functional group to be linked to a drug so as to be linked to the drug afterward (through, for example, click chemistry, a reaction between a sulfhydryl group and a maleimide group, a reaction between an amino group and a succinimidyl group, and the like).

The drug to antibody ratios of an antibody group of the resultant ADC can be analyzed by, for example, known chromatography. When areas of respective peaks in a chromatogram obtained through the analysis of the ADC are calculated, a relative ratio among ADCs having different drug to antibody ratios may be obtained.

The method of the present invention may further comprise

(d) purifying the ADC obtained in the step (c). Purification of the ADC can be performed by a known method. The purification of the ADC can be performed using, for example, an ion exchange column, a hydrophobic interaction column, a gel filtration column, a desalting column, or ultrafiltration.

The method of the present invention may further comprise

(e) adding a pharmaceutically acceptable excipient to the purified ADC.

Examples of the pharmaceutically acceptable excipient include a salt, a buffer, a filler, a chelating agent, an antioxidant, an isotonicity agent, a diluent, a stabilizer, a surfactant (such as a non-ionic surfactant), and a preservative.

The ADC obtained by the method of the present invention may be sterilized by filtration sterilization or the like. The ADC obtained by the method of the present invention may be provided in the form of a freeze-dried formulation (for example, in the form of a combination or a kit of a freeze-dried formulation and a diluent), or in the form of a liquid (for example, in the form of a syringe filled with the ADC in an amount suitable for a single dose). Accordingly, the method of the present invention may further comprise

(f) providing a combination or a kit of a freeze-dried formulation comprising the ADC and a diluent, or a syringe or a vial filled with a liquid comprising the ADC.

The present invention provides a method for increasing yield of an ADC having a drug to antibody ratio of a predetermined value in production of an ADC comprising an antibody and a drug linked to each other via a linker, or a pharmaceutical comprising the ADC, the method comprising

(b) mixing, using a microreactor, a solution comprising tricarboxyethyl phosphine (TCEP) and a partially reduced IgG antibody under reduction reaction with TCEP, with a solution comprising a stoichiometrically excessive amount of an inhibitor of TCEP based on TCEP.

The method of the present invention in this aspect may further comprise

(a) mixing a solution comprising an IgG antibody with a solution comprising tricarboxyethyl phosphine (TCEP) to generate an antibody having a predetermined number of SH groups {wherein the mixing may be performed using or not using a microreactor, and can be preferably performed using a microreactor}.

The method of the present invention in this aspect may further comprise

(c) reacting the partially reduced antibody with a linker having a functional group reactive with an SH group of the antibody to generate the antibody linked to the linker.

The respective steps (a) to (c) of the present invention in this aspect are the same as the steps (a) to (c) of the above-described production method.

In the present invention, the predetermined number may be 1 to 7, may be 2 to 6, may be 3 to 4, or may be 4.

A compound used in the method of the present invention may be provided in a state in which a particle clogging a channel of a microreactor has been removed. The present invention provides a filtered composition used for reducing an antibody, comprising TCEP. The present invention provides a filtered composition used for stopping reduction of an antibody, comprising an inhibitor of TCEP (in particular, preferably one or more inhibitors selected from the group consisting of 4-azidobenzoic acid and 2-azidoethyl-2-acetamido-2-deoxy-β-D-glucopyranoside, and more preferably 2-azidoethyl-2-acetamido-2-deoxy-β-D-glucopyranoside). The present invention provides a filtered composition used for stopping, using a microreactor, reduction of an antibody, comprising an inhibitor of TCEP (in particular, preferably one or more inhibitors selected from the group consisting of 4-azidobenzoic acid and 2-azidoethyl-2-acetamido-2-deoxy-β-D-glucopyranoside, and more preferably 2-azidoethyl-2-acetamido-2-deoxy-β-D-glucopyranoside) .

The present invention provides a use of an inhibitor of TCEP (in particular, preferably one or more inhibitors selected from the group consisting of 4-azidobenzoic acid and 2-azidoethyl-2-acetamido-2-deoxy-β-D-glucopyranoside, and more preferably 2-azidoethyl-2-acetamido-2-deoxy-β-D-glucopyranoside) for stopping, using a microreactor, reduction of an antibody. The present invention provides a use of an inhibitor of TCEP (in particular, preferably one or more inhibitors selected from the group consisting of 4-azidobenzoic acid and 2-azidoethyl-2-acetamido-2-deoxy-β-D-glucopyranoside, and more preferably 2-azidoethyl-2-acetamido-2-deoxy-β-D-glucopyranoside) for increasing yield of an ADC having a drug to antibody ratio of a predetermined value in production, using a microreactor, of an ADC comprising an antibody and a drug linked to each other via a linker, or a pharmaceutical comprising the ADC.

All the production methods of the present invention are applicable to production of an ADC on a commercial scale. The commercial scale means a production scale of an ADC as a pharmaceutical, and may be a treatment of an antibody (in an amount of, for example, about 1 kg to about 10 kg or more) to be produced in a culture solution, in an amount of about 1000 L to about 10000 L or more, containing an antibody producing cell (such as an ovarian cell of a Chinese hamster).

[Examples]

Example 1: Production of Antibody-Drug Conjugate (ADC) (Batch Method)

In this example, a drug was conjugated to an antibody by a batch method.

A drug can be linked to an antibody by reacting an SH group obtained through reduction of a cysteine residue of the antibody and a substituent of a linker linked to the drug. In this example, a scheme for producing an ADC by reacting a maleimide group of a linker linked to a drug with an SH group of an antibody was employed.

In this example, a human monoclonal IgG1 antibody was used as the antibody. An IgG1 antibody is a subclass frequently used in an ADC, and has 4 disulfide bonds between chains as described above. Besides, in this example, monomethyl auristatin D (MMAD), that is, an anticancer agent frequently used in an ADC, was used as the drug. Furthermore, maleimide caproyl-Val-Ala-p-amino-benzoyloxycarbonyl-MMAD (MC-VA-PAB-MMAD) linked to the drug was used as the linker linked to a drug. This linker generates a covalent bond via an SH group of a reduced antibody and the maleimide group.

A reactor was charged with 0.5 mL of a reaction solution (125 mM Tris, 6.25 mM EDTA, 37.5 mM histidine, 75 mM arginine, pH 7.2) containing 20.625 mg of an antibody A (IgG1, Kappa), and the resultant was heated to 35°C. To the resultant, a reducing agent, 10 mM tricarboxyethyl phosphine (TCEP), was added in an amount of 2.1-fold molar equivalent with respect to the antibody to perform a reduction reaction for 15 minutes under stirring. Next, dimethylacetamide (DMA) containing MC-VA-PAB-MMAD (Levena Biopharma, San Diego, CA) in a concentration of 10 mM was added thereto in an amount of 5-fold molar equivalent with respect to the antibody, followed by stirring at 35°C for performing a conjugation reaction for 15 minutes. After completing the reaction, 30 mM N-acetylcysteine (Fujifilm Wako Pure Chemical Corporation) was added thereto in an amount of 5-fold molar equivalent with respect to the antibody, followed by stirring for 1 minute to stop the reaction between the antibody and the linker, and thus, an antibody-drug conjugate (ADC) was obtained. Regarding a general synthesis method for an ADC using TCEP, see, for example, Katherine R. Kozak et al., 2013, Bioconjugate Chemistry, 24: 772-779.

Next, the thus obtained ADC was purified. A desalting column SpinOUT-GT1200, 3 mL (GBioscience) was put in a 15 mL tube, and 3 mL of a buffer (10 mM histidine, 7.5% w/v sucrose, 0.08% w/v Polysorbate 20, pH 5.2) was added thereto repeatedly five times for equilibration. After removing the buffer, the desalting column was put in a new 15 mL tube, 0.5 mL of the ADC obtained as described above was added thereto, and the resultant was centrifuged at 1000 g for 6 minutes to obtain a purified ADC (filtration fraction).

Example 2: Production of Antibody-Drug Conjugate (ADC) (Microreactor Method)

In this example, an ADC was synthesized using a microreactor.

As the microreactor, one described in WO2017-179353 (A1) including a joining channel for joining two channels (supply channels), and designed in such a manner that solutions respectively introduced from ends of the two channels are mixed in the joining channel was used. The microreactor was of a sheath flow type. A plurality of such microreactors were linked in tandem, so as to mix an antibody and a reducing agent in a first microreactor, to mix a reduced antibody and a linker linked to a drug in a second microreactor, and to further mix a regent for stopping a reaction between the antibody and the drug in a third microreactor. The microreactors were linked through tubes, and were designed so that the reaction proceeds also within the tubes. Each of the channels and reaction channels of the microreactors used in this example had a width of 0.2 mm and a depth of 0.2 mm.

An antibody solution (pH 7.2, 35°C) prepared in the same manner as in Example 1 and a reducing agent, 10 mM TCEP, were introduced through the respective channels of the microreactor respectively at flow rates of 2 mL/min and 0.12 mL/min (2.1-fold molecular equivalent based on the antibody) to be mixed in the reaction channel, and the thus obtained mixture was subjected to a reduction reaction for 15 minutes in the tube connected to the microreactor. The resultant reaction solution was successively mixed, using the microreactor, with 10 mM MC-VA-PAB-MMAD at a flow rate of 0.29 mL/min (5-fold molecular equivalent based on the antibody), and the thus obtained mixture was subjected to a conjugation reaction for 15 minutes within the tube. After completing the reaction, the resultant reaction solution was immediately sampled, 30 mM N-acetylcysteine (Fujifilm Wako Pure Chemical Corporation) was added thereto in an amount of 5-fold molecular equivalent based on the antibody, followed by stirring for 1 minute, and thus, an ADC was obtained. It is noted that the microreactors and the tubes were heated to 35°C in a water bath for performing the above-described reactions. The ADC was purified in the same manner as in Example 1.

Example 3: Production of Antibody-Drug Conjugate (ADC) (Microreactor Method)

In this example, an ADC was synthesized using a microreactor.
As the microreactor, the same microreactor as that described in Example 2 except that a channel and a reaction channel had a width of 0.5 mm and a depth of 0.5 mm was used.
An antibody solution (pH 7.2, 35°C) prepared in the same manner as in Example 1 and a reducing agent, 10 mM TCEP, were mixed, using the microreactor, respectively at flow rates of 22 mL/min and 1.3 mL/min (2.1-fold molecular equivalent based on the antibody), and the thus obtained mixture was reacted for 15 minutes in the tube. The resultant reaction solution was successively mixed, using the microreactor, with 10 mM MC-VA-PAB-MMAD at a flow rate of 3.1 mL/min (5-fold molecular equivalent based on the antibody), and the thus obtained mixture was reacted for 15 minutes within the tube. The reduction and conjugation were performed at 35°C. Subsequently, the reaction solution was mixed, using the microreactor, with 30 mM N-acetylcysteine at a flow rate of 1.0 mL/min (5-fold molecular equivalent based on the antibody), and the resultant mixture was subjected to a quenching reaction for 1 minute within the tube, and thus, an ADC was obtained. The ADC was purified in the same manner as in Example 1.

Example 4: Analysis of Drug to Antibody Ratio (DAR) in ADC

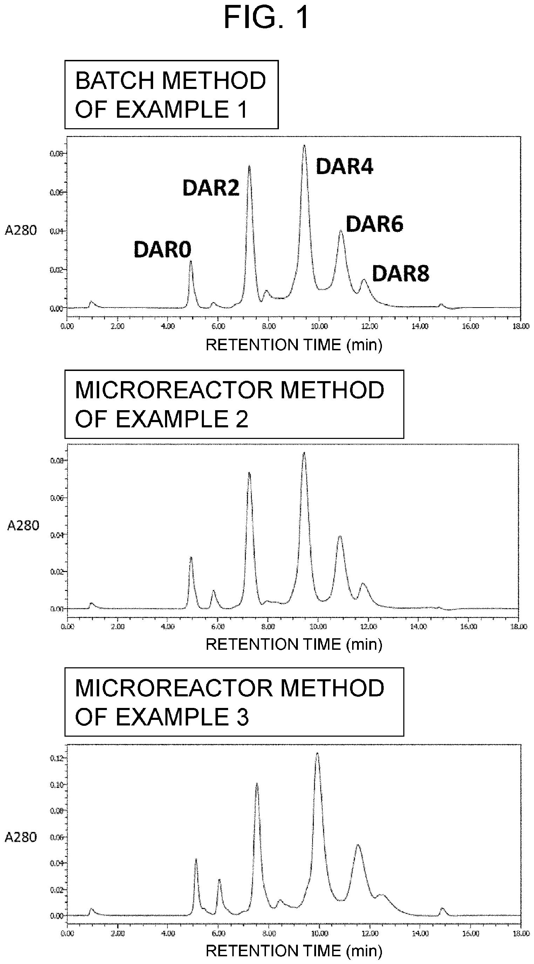

A drug to antibody ratio (DAR) in the obtained ADC was analyzed as follows. A hydrophobic chromatography column, TSKgel Butyl-NPR column (4.6 mm I. D. x 10 cm, 2.5 $\mu$m, Tosoh Bioscience LLC, Japan), was connected to Waters alliance HPLC system for performing analysis with a gradient from a solution A (25 mM sodium phosphate monobasic monohydrate/1.5 M ammonium sulfate, pH 7) to a solution B (75% 25 mM sodium phosphate monobasic monohydrate, pH 7/25% IPA). A peak was detected with UV at 280 nm, and based on area values of peaks thus obtained, each DAR value and an average drug to antibody ratio (average DAR; Ave. DAR) were calculated.
Ave. DAR was determined by multiplying a drug to antibody ratio (0, 1, 2, 3, 4, 6, or 8) of each peak by a peak area %, and dividing a sum of ADC products by 100 (wherein the peak area % refers to a peak area percentage determined depending upon a measured area below a peak of an optical density at 280 nm of UV plotted with respect to retention time (min)).
Chromatograms of the ADCs obtained in Examples 1 to 3 are illustrated in Figure 1. As illustrated in Figure 1, when the batch method was employed, peaks corresponding to the drug to antibody ratios, DARs, of 0, 2, 4, 6 and 8 were observed. Also in the method using the microreactor, peaks corresponding to the drug to antibody ratios, DARs, of 0, 2, 4, 6 and 8 were observed.
Besides, the analysis results of the DARs of the ADCs obtained in Examples 1 to 3 are shown in Table 1.
[Table 1]

Table 1: Results of DAR Analysis of ADCs obtained in Examples 1 to 3

|  | Batch Method of Example 1 | Microreactor Method of Example 2 | Microreactor Method of Example 3 |
|---|---|---|---|
| Ave. DAR | 4.2 | 3.8 | 3.9 |
| DAR 0 | 3.9% | 7.0% | 5.3% |
| DAR 1 | 4.6% | 1.2% | 3.8% |
| DAR 2 | 16.4% | 24.8% | 19.8% |
| DAR 3 | 3.8% | 3.8% | 3.9% |
| DAR 4 | 39.6% | 36.6% | 39.1% |
| DAR 6 | 23.3% | 19.2% | 21.5% |
| DAR 8 | 8.5% | 7.4% | 6.6% |
| * The Ave. DAR was calculated, in employing the batch method, in accordance with the following equation: $\{(0 \times 3.9) + (1 \times 4.6) + (2 \times 16.4) + (3 \times 3.8) + (4 \times 39.6) + (6 \times 23.3) + (8 \times 8.5)\}/100 = 4.2$. | | | |

As shown in Table 1, with respect to the Ave. DAR and a ratio among the peaks of the DARs, equivalent results were obtained in employing the microreactor method to those obtained in employing the batch method.

This reveals that an ADC could be synthesized by employing the microreactor method in the same manner as in employing the batch method. Besides, particularly in yields of ADCs having DARs of 3 to 4, there was no large difference between the batch method and the microreactor method.

Example 5: Production of ADC (Microreactor Method)

As the microreactor, the microreactor described in Example 2 was used.

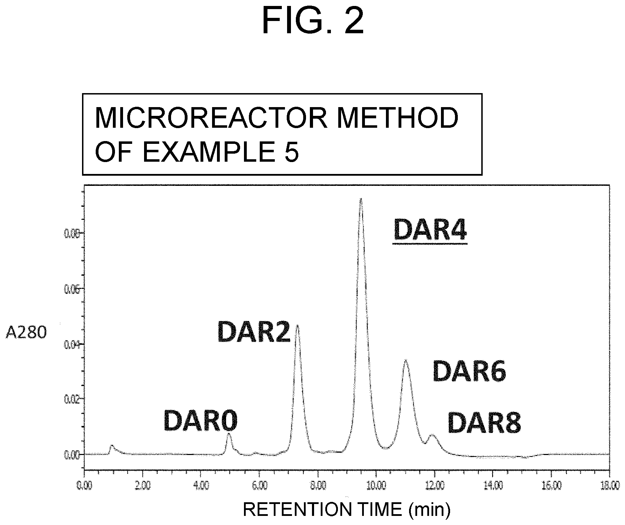

An antibody solution (pH 7.2, 35°C) (prepared by adding 10 mL of a buffer (25 mM EDTA, 0.5 M Tris, pH 7.8) to an antibody solution (30 mL of 27.5 mg/mL of the same antibody used in Example 1, 30 mM histidine, 50 mM arginine, 3.8% w/v sucrose, 0.04% w/v polysorbate 20), and a reducing agent, 10 mM TCEP, were mixed, using the microreactor, respectively at flow rates of 2 mL/min and 0.29 mL/min (10-fold molecular equivalent based on the antibody), and a liquid having passed was reacted at room temperature for 1.5 minutes within the tube. The resultant reaction solution was successively mixed, using the microreactor, with a mixture of 14 mM MC-VA-PAB-MMAD and 70 mM 4-azidobenzoic acid (4-ABA), that is, an inhibitor of TCEP, at a flow rate of 0.2 mL/min (MC-VA-PAB-MMAD in an amount of 10-fold molar equivalent based on the antibody, and 4-ABA in an amount of 50-fold molar equivalent based on the antibody) to perform a reaction at room temperature for 30 minutes within the tube. After completing the conjugation reaction, the resultant reaction solution was sampled, 30 mM N-acetylcysteine (Fujifilm Wako Pure Chemical Corporation) was added thereto in an amount of 5-fold molar equivalent based on the antibody, followed by stirring for 1 minute to perform a quenching reaction, and thus, an ADC was obtained. The ADC was purified in the same manner as in Example 1, and DAR values were analyzed in the same manner as in Example 4. The thus obtained chromatogram is illustrated in Figure 2, and analysis results of DARs are shown in Table 2.

As illustrated in Figure 2, when the microreactor method of Example 5 was employed, a peak of an ADC having a DAR of 4 was relatively larger than peaks of the other ADCs having different DARs.

[Table 2]

Table 2: Effect of Microreactor Method of Example 5 on DAR

|  | Batch Method of Example 1 | Microreactor Method of Example 2 | Microreactor Method of Example 3 | Microreactor Method of Example 5 |
|---|---|---|---|---|
| Ave. DAR | 4.2 | 3.8 | 3.9 | 4.2 |
| DAR 0 | 3.9% | 7.0% | 5.3% | 2.6% |
| DAR 1 | 4.6% | 1.2% | 3.8% | 1.4% |
| DAR 2 | 16.4% | 24.8% | 19.8% | 18.3% |
| DAR 3 | 3.8% | 3.8% | 3.9% | 0.0% |
| DAR 4 | 39.6% | 36.6% | 39.1% | 47.1% |
| DAR 6 | 23.3% | 19.2% | 21.5% | 25.4% |
| DAR 8 | 8.5% | 7.4% | 6.6% | 5.3% |

As shown in Table 2, in employing the microreactor method of Example 5, as compared with the methods employed in Examples 1 to 3, for example, a ratio of an ADC having a DAR of 4 was significantly larger than a ratio of an ADC having another DAR. According to Table 2, although the ratio of the ADC having a DAR of 4 could not be largely varied by mixing TCEP using a microreactor, a distribution of the DARs was largely varied when the reduction of the antibody was stopped by mixing, using a microreactor, the inhibitor of TCEP in an excessive amount based on the antibody under reduction reaction. Accordingly, it was revealed that the mixing of a reduction inhibitor with an antibody under reduction using a microreactor affects a DAR distribution and may improve the yield of the ADC having a DAR of 4. Besides, in the microreactor described in Example 5, the channels were not clogged after completing the reaction.

Example 6: Production of Antibody-Drug Conjugate (ADC) (Batch Method)

In this example, a drug was conjugated to an antibody by employing the batch method.

A drug can be linked to an antibody by reacting an SH group obtained by reducing a cysteine residue of the antibody with a substituent of a linker linked to the drug. In this example, a scheme for producing an ADC by reacting a maleimide group of a linker linked to a drug with an SH group of an antibody was employed.

In this example, a human monoclonal IgG1 antibody (Herceptin) was used as the antibody. An IgG1 antibody is a subclass frequently used in an ADC, and has 4 disulfide bonds between chains as described above. Besides, in this example, monomethyl auristatin D (MMAD), that is, an anticancer agent frequently used in an ADC, was used as the drug. Furthermore, maleimide caproyl-Val-Ala-p-amino-benzoyloxycarbonyl-MMAD (MC-VA-PAB-MMAD) linked to the drug was used as the linker linked to a drug. This linker generates a covalent bond via an SH group of a reduced antibody and the maleimide group.

A reactor was charged with 0.5 mL of a reaction solution (125 mM Tris, 6.25 mM EDTA, 37.5 mM histidine, 75 mM arginine) containing 10.15 mg of Herceptin. To the resultant, a reducing agent, 10 mM tricarboxyethyl phosphine (TCEP), was added in an amount of 2.4-fold molar equivalent with respect to the antibody to perform a reduction reaction for 60 minutes under stirring. Next, dimethylacetamide (DMA) containing MC-VA-PAB-MMAD (Levena Biopharma, San Diego, CA) in a concentration of 10 mM was added thereto in an amount of 5-fold molar equivalent with respect to the antibody to perform a conjugation reaction for 60 minutes. After completing the reaction, 30 mM N-acetylcysteine (Fujifilm Wako Pure Chemical Corporation) was added thereto in an amount of 10-fold molar equivalent with respect to the antibody, followed by stirring for 1 minute to stop the reaction between the antibody and the linker, and thus, an antibody-drug conjugate (ADC) was obtained. Regarding a general synthesis method for an ADC using TCEP, see, for example, Katherine R. Kozak et al., 2013, Bioconjugate Chemistry, 24: 772-779.

Next, the thus obtained ADC was purified. A desalting column SpinOUT-GT1200, 3 mL (GBioscience) was put in a 15 mL tube, and 3 mL of a buffer (10 mM histidine, 7.5% w/v sucrose, 0.08% w/v Polysorbate 20, pH 5.2) was added thereto repeatedly five times for equilibration. After removing the buffer, the desalting column was put in a new 15 mL tube, 0.5 mL of the ADC obtained as described above was added thereto, and the resultant was centrifuged at 1000 g for 6 minutes to obtain a purified ADC (filtration fraction).

Example 7: Production of ADC (Microreactor Method)

As the microreactor, the same microreactor as that used in Example 2 was used.

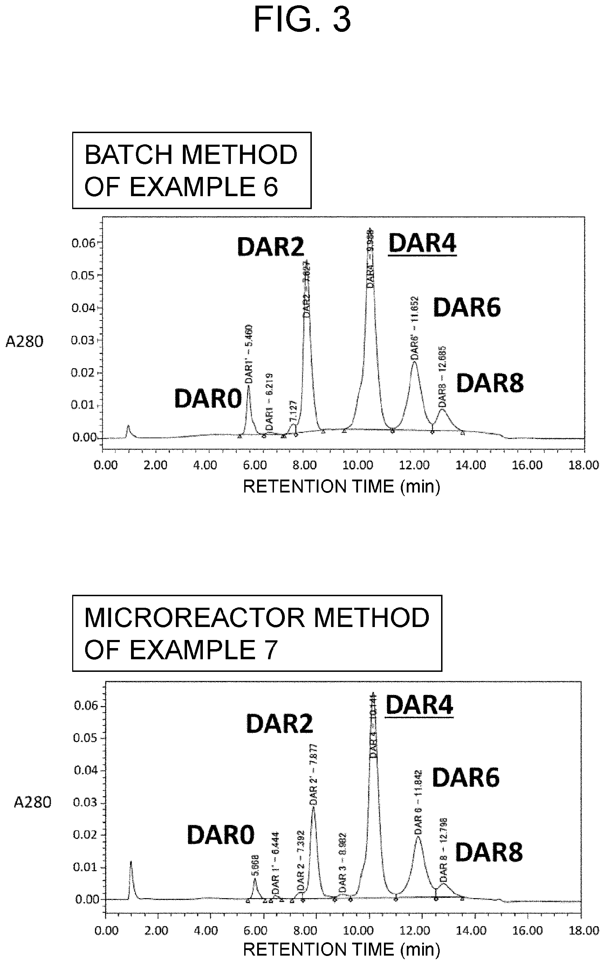

An antibody solution (prepared by adding 20 mL of a buffer (25 mM EDTA, 0.5 M Tris, pH 7.8) to 60 mL of an antibody solution (20.3 mg/mL of Herceptin, 30 mM histidine, 50 mM arginine, 3.8% w/v sucrose, 0.04% w/v polysorbate 20), and a reducing agent, 10 mM TCEP, were mixed, using the microreactor, respectively at flow rates of 1.5 mL/min and 0.15 mL/min (10-fold molecular equivalent based on the antibody), and a liquid having passed was reacted at room temperature for 2 minutes within the tube. The resultant reaction solution was successively mixed, using the microreactor, with a mixture of 14 mM MC-VA-PAB-MMAD and 70 mM 4-azidobenzoic acid (4-ABA), that is, an inhibitor of TCEP, at a flow rate of 0.11 mL/min (MC-VA-PAB-MMAD in an amount of 10-fold molar equivalent based on the antibody, and 4-ABA in an amount of 50-fold molar equivalent based on the antibody) to perform a reaction at room temperature for 35 minutes within the tube. After completing the conjugation reaction, the resultant reaction solution was sampled, 30 mM N-acetylcysteine (Fujifilm Wako Pure Chemical Corporation) was added thereto in an amount of 5-fold molar equivalent based on the antibody, followed by stirring for 1 minute to perform a quenching reaction, and thus, an ADC was obtained. The ADC was purified in the same manner as in Example 1, and DAR values were analyzed in the same manner as in Example 4. The thus obtained chromatogram is illustrated in Figure 3, and analysis results of DARs are shown in Table 3.

[Table 3]

Table 3: Results of DAR Analysis of ADCs obtained in Examples 6 and 7

|  | Batch Method of Example 6 | Microreactor Method of Example 7 |
|---|---|---|
| Ave. DAR | 3.9 | 4.1 |
| DAR 0 | 4.6% | 2.1% |
| DAR 1 | 0.3% | 0.3% |

(continued)

|  | Batch Method of Example 6 | Microreactor Method of Example 7 |
|---|---|---|
| DAR 2 | 26.2% | 17.9% |
| DAR 3 | 0.0% | 0.9% |
| DAR 4 | 45.2% | 52.8% |
| DAR 6 | 18.4% | 21.8% |
| DAR 8 | 5.4% | 4.3% |

As illustrated in Figure 3, in employing the microreactor method of Example 7, a peak of an ADC having a DAR of 4 was relatively larger than peaks of the other ADCs having different DARs as compared with that obtained by the batch method of Example 6.

As shown in Table 3, in employing the microreactor method of Example 7, as compared with the method employed in Example 6, for example, a ratio of an ADC having a DAR of 4 was significantly larger than a ratio of an ADC having another DAR. According to Table 3, a distribution of the DARs was largely changed when the reduction of the antibody was stopped by mixing, using a microreactor, the inhibitor of TCEP in an excessive amount based on the antibody under reduction reaction. Accordingly, it was revealed that the mixing of a reduction inhibitor with an antibody under reduction using a microreactor affects a DAR distribution and may improve the yield of the ADC having a DAR of 4. Although different antibodies were used in Example 5 and Example 7, it was revealed that the DAR distribution is changed by excessive mixture of the inhibitor of TCEP in using either of the antibodies. Besides, in the microreactor described in Example 7, the channels were not clogged after completing the reaction.

Example 8: Production of ADC (Microreactor Method)

As the microreactor, a microreactor Spica (static type) manufactured by YMC including a joining channel for joining two channels (supply channels), and designed in such a manner that solutions respectively introduced from ends of the two channels are mixed in the joining channel was used. A plurality of such microreactors were linked in tandem, so as to mix an antibody and a reducing agent in a first microreactor, and to mix a reduced antibody, a linker linked to a drug and a TCEP inhibitor in a second microreactor. The microreactors were linked through a tube, and were designed so that the reaction proceeds also within the tube. Each of the channels of the microreactors used in this example had a width of 0.2 mm and a depth of 0.2 mm.

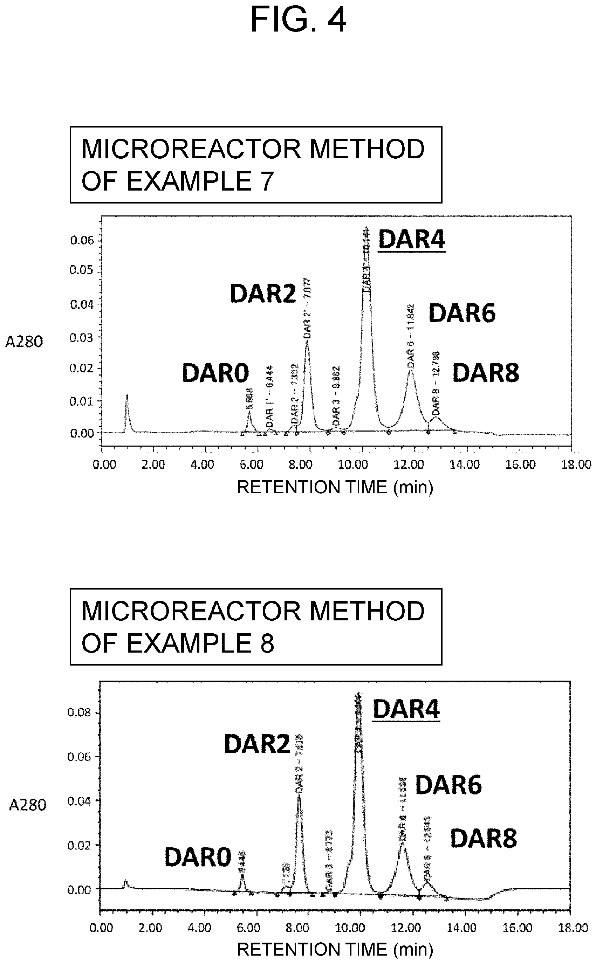

An antibody solution prepared by adding 20 mL of a buffer (25 mM EDTA, 0.5 M Tris, pH 7.8) to 60 mL of an antibody solution (20.3 mg/mL Herceptin, 30 mM histidine, 50 mM arginine, 3.8% w/v sucrose, 0.04% w/v polysorbate 20), and a reducing agent, 10 mM TCEP, were mixed, using the microreactors, respectively at flow rates of 1.5 mL/min and 0.15 mL/min (10-fold molar equivalent based on the antibody), and a liquid having passed was reacted at room temperature for 2 minutes within the tube. The resultant reaction solution was successively mixed, using the microreactor, with a mixture of 14 mM MC-VA-PAB-MMAD and an inhibitor of TCEP, 70 mM 4-azidobenaoic acid (4-ABA) at a flow rate of 0.11 mL/min (MC-VA-PAB-MMAD in an amount of 10-fold molecular equivalent based on the antibody, and 4-ABA in an amount of 50-fold molecular equivalent based on the antibody) to perform a reaction at room temperature for 35 minutes within the tube. After completing the conjugation reaction, the resultant reaction solution was sampled, 30 mM N-acetylcysteine (Fujifilm Wako Pure Chemical Corporation) was added thereto in an amount of 5-fold molar equivalent based on the antibody, followed by stirring for 1 minute to perform a quenching reaction, and thus, an ADC was obtained. The ADC was purified in the same manner as in Example 1, and DAR values were analyzed in the same manner as in Example 4. The thus obtained chromatogram is illustrated in Figure 4, and analysis results of DARs are shown in Table 4.

[Table 4]

Table 4: Effect of Microreactor Methods of Examples 7 and 8 on DAR

|  | Batch Method of Example 6 | Microreactor Method of Example 7 | Microreactor Method of Example 8 |
|---|---|---|---|
| Ave. DAR | 3.9 | 4.1 | 4.2 |
| DAR 0 | 4.6% | 2.1% | 1.8% |
| DAR 1 | 0.3% | 0.3% | 0.0% |
| DAR 2 | 26.2% | 17.9% | 18.6% |

(continued)

|  | Batch Method of Example 6 | Microreactor Method of Example 7 | Microreactor Method of Example 8 |
|---|---|---|---|
| DAR 3 | 0.0% | 0.9% | 0.3% |
| DAR 4 | 45.2% | 52.8% | 53.9% |
| DAR 6 | 18.4% | 21.8% | 20.7% |
| DAR 8 | 5.4% | 4.3% | 4.8% |

As illustrated in Figure 4, in employing the microreactor method of Example 8, in the same manner as in the microreactor method of Example 7, a peak of an ADC having a DAR of 4 was relatively larger than peaks of the other ADCs having different DARs as compared with that obtained by the batch method of Example 6.

In Example 7 and Example 8, the results of performing excessive mixture of the TCEP inhibitor using the different microreactors are shown. As shown in Table 4, it was revealed, through comparison between the microreactor methods of Example 7 and Example 8, that ADC compounds having equivalent DARs are obtained. Besides, in the microreactor described in Example 8, the channels were not clogged after completing the reaction.

Example 9: Production of ADC (Microreactor Method)

As the microreactor, the same microreactor as that used in Example 7 was used. Each of the channels of the microreactor used in this example had a width of 0.2 mm and a depth of 0.2 mm.

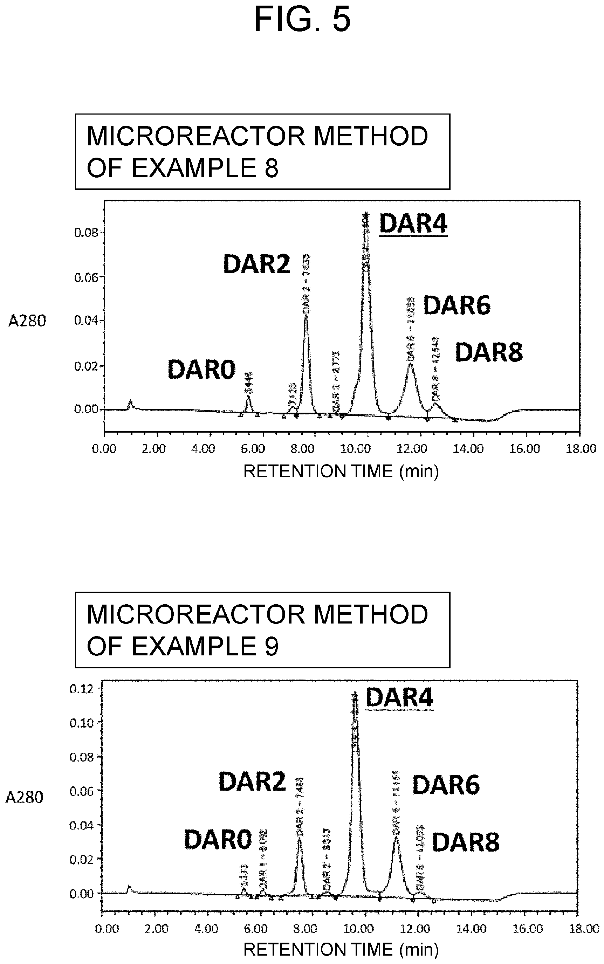

An antibody solution prepared by adding 19.7 mL of a buffer (25 mM EDTA, 0.5 M Tris, pH 7.8) to 59 mL of an antibody solution (20.3 mg/mL Herceptin, 30 mM histidine, 50 mM arginine, 3.8% w/v sucrose, 0.04% w/v polysorbate 20), and a reducing agent, 10 mM TCEP, were mixed, using the microreactor, respectively at flow rates of 1.5 mL/min and 0.11 mL/min (7-fold molar equivalent based on the antibody), and a liquid having passed was reacted at room temperature for 10 seconds within the tube. The resultant reaction solution was successively mixed, using the microreactor, with a mixture of 14 mM MC-VA-PAB-MMAD and an inhibitor of TCEP, 140 mM 2-azidoethyl-2-acetamido-2-deoxy-β-D-glucopyranoside (AADG) at a flow rate of 0.11 mL/min (MC-VA-PAB-MMAD in an amount of 10-fold molecular equivalent based on the antibody, and 4-ABA in an amount of 100-fold molecular equivalent based on the antibody) to perform a reaction at room temperature for 20 minutes within the tube. After completing the conjugation reaction, the resultant reaction solution was sampled, 30 mM N-acetylcysteine (Fujifilm Wako Pure Chemical Corporation) was added thereto in an amount of 5-fold molar equivalent based on the antibody, followed by stirring for 1 minute to perform a quenching reaction, and thus, an ADC was obtained. The ADC was purified in the same manner as in Example 1, and DAR values were analyzed in the same manner as in Example 4. The thus obtained chromatogram is illustrated in Figure 5, and analysis results of DARs are shown in Table 5.

[Table 5]

Table 5: Effect of Microreactor Methods of Examples 8 and 9 on DAR

|  | Batch Method of Example 6 | Microreactor Method of Example 8 | Microreactor Method of Example 9 |
|---|---|---|---|
| Ave. DAR | 3.9 | 4.2 | 4.2 |
| DAR0 | 4.6% | 1.8% | 0.8% |
| DAR 1 | 0.3% | 0.0% | 0.8% |
| DAR 2 | 26.2% | 18.6% | 12.6% |
| DAR 3 | 0.0% | 0.3% | 1.2% |
| DAR 4 | 45.2% | 53.9% | 59.2% |
| DAR 6 | 18.4% | 20.7% | 23.3% |
| DAR 8 | 5.4% | 4.8% | 2.1% |

As illustrated in Figure 5, in employing the microreactor method of Example 9, a peak of an ADC having a DAR of 4 was relatively larger than peaks of the other ADCs having different DARs as compared with that obtained by the microreactor method of Example 8.

As shown in Table 5, in employing the microreactor method of Example 9, as compared with the microreactor method employed in Example 8, for example, a ratio of an ADC having a DAR of 4 was significantly larger than a ratio of an ADC having another DAR. According to Table 5, it was revealed that the azide compounds used as the TCEP reducing agent, that is, 4-azidobenzoic acid (4-ABA) and 2-azidoethyl-2-acetamide-2-deoxy-β-D-glucopyranoside (AADG), both have an effect of increasing the ratio of DAR4. Besides, it was revealed that 2-azidoethyl-2-acetamide-2-deoxy-β-D-glucopyranoside (AADG) has a higher effect of increasing the ratio of DAR4 than 4-azidobenzoic acid (4-ABA). Furthermore, in the microreactor described in Example 9, the channels were not clogged after completing the reaction.

In this manner, the following was revealed: An antibody is reduced with a reducing agent to obtain a partially reduced antibody. The partially reduced antibody and an inhibitor of the reducing agent are mixed in a microreactor including a microchannel and a mixing channel to stop the reduction reaction of the antibody at an appropriate timing. Thus, the extent of a reduction state of the antibody can be controlled. Therefore, a linker having a group reactive with an SH group and a payload (drug) bond to the antibody in a ratio in accordance with the reduction state. Accordingly, in employing the methods exemplarily described in the examples, the yield of an ADC having a desired DAR can be increased. Although the methods for increasing production efficiency of an ADC having a DAR of 4 have been exemplarily described in the examples, the DAR would be able to be adjusted to another value different from 4. For example, the method of the present invention may be employed for obtaining a DAR distribution suitable for each IgG subtype. It was also revealed that any of various reduction inhibitors can be used as the reduction inhibitor, and that a reduction inhibitor may be appropriately selected therefrom in accordance with a purpose of a product. In the examples, it was revealed that 4-ABA and AADG are useful in the method for improving the production efficiency of an ADC. In the present invention, it was revealed that a microreactor is useful in process for mixing a solution for stopping a reduction reaction with an antibody solution under reduction reaction. Besides, in the present invention, it was revealed that a microreactor is useful in process for mixing a solution for starting a reduction reaction with an antibody solution.

Industrial Applicability

Inhibition or a stop of a reduction reaction of an antibody using a microreactor may be useful as a method for controlling or changing a DAR value of an ADC (in particular, a method for producing an ADC having a DAR of 4, or a method for improving the yield of an ADC having a DAR of 4). The production method of the present invention may be useful as a production method for an ADC on a commercial scale.

**Claims**

1. A method for producing an antibody-drug conjugate (ADC) comprising an antibody and a drug linked to each other via a linker, or a pharmaceutical comprising the ADC, the method comprising
mixing, using a microreactor, a solution comprising a reducing agent for reducing a disulfide bond of the antibody and a partially reduced IgG antibody under reduction reaction with the reducing agent, with a solution comprising an inhibitor of the reducing agent and/or a reduction terminator.

2. A method for producing an antibody-drug conjugate (ADC) comprising an antibody and a drug linked to each other via a linker, or a pharmaceutical comprising the ADC, the method comprising:

   (b) mixing, using a microreactor, a solution comprising tricarboxyethyl phosphine (TCEP) and a partially reduced IgG antibody under reduction reaction with TCEP, with a solution comprising a stoichiometrically excessive amount of an inhibitor of TCEP based on TCEP.

3. The method according to claim 2, further comprising:

   (a) mixing, using a microreactor, a solution comprising an IgG antibody with a solution comprising tricarboxyethyl phosphine (TCEP) to generate a partially reduced antibody.

4. The method according to claim 2 or 3, further comprising:

   (c) reacting the partially reduced antibody with a linker having a functional group reactive with an SH group of the antibody to generate the antibody linked to the linker.

5. A method for producing an antibody-drug conjugate (ADC) comprising an antibody and a drug linked to each other via a linker, or a pharmaceutical comprising the ADC, the method comprising:

(a) mixing, using a microreactor, a solution comprising an IgG antibody with a solution comprising tricarboxyethyl phosphine (TCEP) to generate a partially reduced antibody;
(b) mixing, using a microreactor, a solution comprising tricarboxyethyl phosphine (TCEP) and the partially reduced IgG antibody under reduction reaction with TCEP with a solution comprising a stoichiometrically excessive amount of an inhibitor of TCEP based on TCEP; and
(c) reacting the partially reduced antibody with a linker having a functional group reactive with an SH group of the antibody to generate the antibody linked to the linker.

6. The method according to any one of claims 2 to 5, wherein the solution comprising the inhibitor of TCEP further comprises the linker.

7. The method according to claim 4 or 5, further comprising, before (c), mixing, using a microreactor, a solution obtained in (b) with a solution comprising the linker.

8. The method according to any one of claims 2 to 7, wherein the inhibitor of TCEP is one or more inhibitors selected from the group consisting of 4-azidobenzoic acid and 2-azidoethyl-2-acetamide-2-deoxy-β-D-glucopyranoside.

9. The method according to any one of claims 2 to 8, wherein the partially reduced antibody is an antibody having four SH groups.

10. The method according to any one of claims 4 to 9, wherein the functional group reactive with an SH group of the antibody is a maleimide group.

11. The method according to any one of claims 4 to 10, wherein the linker is a linker linked to a drug.

12. The method according to any one of claims 4 to 11, wherein the linker is linked to a drug, and the functional group reactive with an SH group of the antibody is a maleimide group.

# FIG. 1

BATCH METHOD
OF EXAMPLE 1

MICROREACTOR METHOD
OF EXAMPLE 2

MICROREACTOR METHOD
OF EXAMPLE 3

# FIG. 2

MICROREACTOR METHOD OF EXAMPLE 5

# FIG. 3

BATCH METHOD OF EXAMPLE 6

MICROREACTOR METHOD OF EXAMPLE 7

# FIG. 4

MICROREACTOR METHOD OF EXAMPLE 7

MICROREACTOR METHOD OF EXAMPLE 8

# FIG. 5

MICROREACTOR METHOD
OF EXAMPLE 8

MICROREACTOR METHOD
OF EXAMPLE 9

## INTERNATIONAL SEARCH REPORT

| International application No. |
| --- |
| PCT/JP2019/040059 |

**A. CLASSIFICATION OF SUBJECT MATTER**
Int.Cl. A61K39/395(2006.01)i, A61K45/00(2006.01)i, A61K47/65(2017.01)i,
A61K47/68(2017.01)i, A61P35/00(2006.01)i, A61P35/02(2006.01)i,
B01J19/00(2006.01)n, C07K16/18(2006.01)n
According to International Patent Classification (IPC) or to both national classification and IPC

**B. FIELDS SEARCHED**

Minimum documentation searched (classification system followed by classification symbols)
Int.Cl. A61K39/395, A61K45/00, A61K47/65, A61K47/68, A61P35/00,
A61P35/02, B01J19/00, C07K16/18

Documentation searched other than minimum documentation to the extent that such documents are included in the fields searched
Published examined utility model applications of Japan    1922–1996
Published unexamined utility model applications of Japan    1971–2019
Registered utility model specifications of Japan    1996–2019
Published registered utility model applications of Japan    1994–2019

Electronic data base consulted during the international search (name of data base and, where practicable, search terms used)
JSTPlus/JMEDPlus/JST7580(JDreamIII), CAplus/MEDLINE/EMBASE/BIOSIS(STN)

**C. DOCUMENTS CONSIDERED TO BE RELEVANT**

| Category* | Citation of document, with indication, where appropriate, of the relevant passages | Relevant to claim No. |
| --- | --- | --- |
| A | DORONINA, S. O. et al., Development of potent monoclonal antibody auristatin conjugates for cancer therapy, Nature Biotechnology, July 2003, 21(7), pp. 778-784, abstract, p. 783, conjugate preparation | 1-12 |
| A | JP 2017-189729 A (HITACHI, LTD.) 19 October 2017, claims, paragraph [0157] & US 2019/0118156 A1, claims, paragraph [0236] & WO 2017/179353 A1 & EP 3444027 A1 | 1-12 |

☒ Further documents are listed in the continuation of Box C.　　☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
| --- | --- |
| 30.10.2019 | 12.11.2019 |

| Name and mailing address of the ISA/ | Authorized officer |
| --- | --- |
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

| International application No. |
| --- |
| PCT/JP2019/040059 |

C (Continuation).    DOCUMENTS CONSIDERED TO BE RELEVANT

| Category* | Citation of document, with indication, where appropriate, of the relevant passages | Relevant to claim No. |
| --- | --- | --- |
| A | JP 2016-531914 A (STEM CENTRX, INC.) 13 October 2016, claims, examples<br>& US 2016/0175460 A1, claims, examples & WO 2015/031693 A1 & EP 3038659 A1 | 1-12 |
| A | JP 2008-501029 A (GENENTECH INC.) 17 January 2008, claims, examples<br>& US 2005/0276812 A1, claims, examples & WO 2005/117986 A2 & EP 1753463 A2 | 1-12 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2005117986 A
- WO 0138318 A
- WO 2017179353 A1

**Non-patent literature cited in the description**

- **DEBAENE F. et al.** *Analytical Chemistry,* 2014, vol. 86, 10674-10683
- **KATHERINE R. KOZAK et al.** *Bioconjugate Chemistry,* 2013, vol. 24, 772-779